# EUROPÄISCHE PATENTSCHRIFT

(11) **EP 3 612 244 B1**
(45) Veröffentlichungstag und Bekanntmachung des Hinweises auf die Patenterteilung: **07.10.2020**
(21) Anmeldenummer: 18748902.6
(22) Anmeldetag: 30.07.2018
(51) Int. Cl.: A61M 1/00, A61M 1/06, F04B 39/00

(54) **MUTTERMILCHPUMPE**
BREAST MILK PUMP
POMPE TIRE-LAIT

(30) Priorität: 22.08.2017 DE 102017007910
(43) Veröffentlichungstag der Anmeldung: 26.02.2020
(73) Patentinhaber: KaWeCo GmbH, D-71254 Ditzingen (DE)
(72) Erfinder: KIRCHNER, Hansjörg, 71706 Markgröningen (DE)
(74) Vertreter: Fleck, Hermann-Josef
(86) Internationale Anmeldenummer: PCT/EP2018/070612
(87) Internationale Veröffentlichungsnummer: WO 2019/038029

(56) Entgegenhaltungen:
- WO-A1-2016/103031
- KR-A- 20140 125 196
- US-A- 4 857 051
- US-A- 4 961 726
- US-A1- 2001 038 799

## Beschreibung

Muttermilchpumpen werden von stillenden Müttern aus verschiedensten Gründen verwendet. Es kommen Handmilchpumpen oder elektrische Milchpumpen zum Einsatz. Letztere bestehen aus einem Pumpset und einer Brustpumpe zum erzeugen des Vakuums. An diese Brustpumpe können auch zwei Pumpsets angeschlossen sein, damit gleichzeitig an beiden Brüsten Muttermilch absaugbar ist. Der Abpumpablauf gliedert sich in zwei Phasen, die Stimulationsphase und die Abpumpphase. Die Stimulationsphase ahmt das zu Beginn schnelle, schwache Saugen des Babys nach, massiert die Brust sanft und regt den Milchfluss an. Die schonende und effektive Abpumpphase simuliert das langsamere, intensivere Saugen des Babys. Dabei wird effektiv die wertvolle Muttermilch gewonnen.

Das Pumpset besteht aus einer Brustglocke, die verschiedene Größen haben kann, einer Milchflasche und einer Schutzmembran, welche die Muttermilchpumpe hermetisch abriegelt. So können weder Bakterien oder andere Keime weder die Schläuche noch die Pumpe kontaminieren.

Die Brustpumpe enthält in ihrem Gehäuse die Druckpumpe für den erforderlichen Luftdruck, die Steuer- und Regelventile zur Beeinflussung des Luftdruckes, die Bedienungsmittel und die Luftdruckleitungen, welche die Pumpsets mit der Brustpumpe verbinden.

Während des Betriebes stellt die Brustpumpe, je nach Betriebsbedingungen, eine als unangenehm wahrgenommene Schallquelle dar.

Die EP 2 260 885 B1 schützt eine Brustpumpe bzw. Milchpumpe, aufweisend eine Brusthaube oder Brustglocke, eine mit dieser verbundene Vakuumquelle und ein vorprogrammiertes Programm zum Betreiben der Vakuumquelle. Die Brustpumpe weist einen Milcheinschussknopf auf, mit dem die Milcheinschusssequenz gemäß der Vorprogrammierung aktiviert wird. Während der Milcheinschusssequenz oder Stimulationsphase wird ein Vakuumbereich von 50 bis etwa 150 mmHg angewandt, bei einer Frequenz von 80 bis 160 Zyklen pro Minute. Nach dieser Stimulationsphase kann mittels Milcheinschussknopf auf die Abpumpphase umgestellt werden. Dabei kommt ein Vakuumbereich von 100 bis 250 mmHg mit einer Frequenz von 47 bis 78 Zyklen pro Minute zur Anwendung.

Bei dieser Ausführung wird das von dem Vakuumerzeuger bzw. der Vakuumquelle erzeugte Vakuum mittels Schlauch zur Brusthaube oder dem Brusthaubentrichter geleitet. Der Vakuumerzeuger besteht aus einem Elektromotor, der über Zahnriemengetriebe auf eine Antriebswelle wirkt, die Stößel, die an Membranen angebracht sind, axial bewegt. Die Membranen erzeugen den Luftdruck mit Hilfe eines Einwegventils. Es realisiert das Prinzip einer drehzahlgeregelten Membranpumpe. Alle diese Bauteile sind in einem gemeinsamen Gehäuse untergebracht.

Weiterhin ist aus der EP 1 843 807 B1 ein Pumpset zum Abpumpen von menschlicher Muttermilch bekannt, welches eine Brusthaube mit einem Brusthaubentrichter zur Anlage an eine Mutterbrust, ein erstes Kopplungsteil zur Verbindung mit einem Muttermilchsammelbehälter und ein zweites Kopplungsteil zur Verbindung mit einer Saugpumpe aufweist. Weiterhin ist ein Bedienungsmittel zur Bedienung der Saugpumpe vorhanden, das im Bereich des Brusthaubentrichters angeordnet ist. Der Fachmann interpretiert die Saugpumpe in dieser Druckschrift als Membranpumpe, wie sie in der EP 2 260 885 B1 dargestellt ist.

Es ist bei Brustpumpen bekannt, dass Vakuumerzeuger mit unterschiedlichen Drehzahlen betrieben werden, und wenn dieser abgeschaltet wird, ein Ventil zum Umgebungsdruck geöffnet wird. Als Beispiel dazu sei die DE 20 2010 014 785 U1 genannt.

Die US 2001/0038799 A1 offenbart eine Muttermilchpumpe bestehend aus aus einem oder zwei Pumpsets, die jeweils mit einem Luftschlauch mit einer Brustpumpe verbunden sind, wobei das Pumpset aus einer Brustglocke, aus einer Milchflasche, die von unten in die Brustglocke eingebracht ist, einem Ventil, das zwischen der Milchflasche und der Brustglocke angeordnet ist, und eine Schutzmembran, die an der Brustglocke angebaut ist und wobei der Verschlussdeckel über einen Schlauchanschluss zum Anschließen des Luftschlauches verfügt, der mit der Brustpumpe verbunden ist, besteht und die Brustpumpe ein Gehäuse aufweist, in dem mindestens eine pneumatische Pumpe und Vakuum regulierende Vorrichtungen eingebaut sind. Das Gehäuse besteht aus zwei Gehäusehälften die beim Zusammenbau eine innere Gehäusewand bilden, wodurch zwei Kammern gebildet werden. Die inneren Gehäusehälften sind mit einem geräuschmindernden und schwingungsmindernden Werkstoff ausgekleidet.

In der einen Kammer ist ein Motor untergebracht, dessen Antriebswelle durchdringt die innere Gehäusewand und treibt einen Exzenter in der anderen Kammer. Dieser bewegt, nach Fig. 14 dieses Standes der Technik, die Kolben, die ihrerseits eine elastische Membran tragen. Verschlussteile sind mit Vakuum regulierenden Vorrichtungen ausgestattet. Diese sind für die Funktion der Vakuumpumpe notwendig und auch begrifflich Bestandteil der Vakuumpumpe.

Bautypisch für diese bekannten Membranpumpen ist ihre Geräuscherzeugung, bedingt durch die mit hoher Frequenz in Abhängigkeit von der Drehzahl des die Membranpumpe antreibenden Elektromotors schwingenden Membran. Auch die Druckschwankungen, wie Überdruck und Unterdruck in den Leitungen von der Brustpumpe hin zum Pumpset, sowie, je nach Ausführung der Ventile, deren Betrieb, stellen eine weitere Geräuschquelle dar. Die ergriffenen Maßnahmen zur Schalldämmung sind aufwendig und im Ergebnis nicht völlig zufriedenstellend.

Diese Nachteile zu vermeiden, ist Aufgabe der vorliegenden Erfindung. Es soll eine geräuscharme Brustpumpe, die ein präzises Vakuum im Anwendungsteil Pumpset bereitstellt, zur Verfügung gestellt werden.

Dies wird mit den Merkmalen des Patentanspruches 1 erreicht. Die Unteransprüche enthalten weitere sinnvolle Ausgestaltungen der Erfindung nach Patentanspruch 1.

Durch Einfügen einer Trennwand in das Gehäuse der Brustpumpe nach Patentanspruch 1 entstehen baulich zwei Räume im Gehäuse. In jeden Raum wird ein mit eigener Dämmung versehenes Kompartiment eingebaut. Diese zwei Kompartimente sind hinsichtlich ihrer Schallschwingungen entkoppelt, was mögliche Resonanzen verhindert.

Nach Patentanspruch 2 enthält das eine Kompartiment die pneumatische Pumpe, die eine spezielle Schallquelle darstellt. Die Dämmung dieses Kompartiments ist baulich und schalltechnisch optimiert, sodass kein hörbarer Schall nach außen dringt. Diese verbesserte Wirkung der Dämmung, die hinsichtlich der kritischen Schallfrequenzen abgestimmt ist, ist deshalb besonders effektiv.

Im zweiten Kompartiment sind, gemäß Patentanspruch 3, die Ventile untergebracht und auch dieses Kompartiment ist mit einer Dämmung versehen, die hier baulich und schalltechnisch hinsichtlich der Schallerzeugung der verwendeten Ventile optimiert ist. Auch dies garantiert eine effektive Schalldämpfung.

Die elektrischen und pneumatischen Verbindungen der Kompartimente nach Patentanspruch 4 verhindern die Übertragung des Körperschalles und stellt eine Entkopplung dar. Resonanzbildung wird so ausgeschlossen.

Patentanspruch 5 spezifiziert die Ventile. Da Luft ein kompressibles Gas ist, sind seine Parameter sinnvollerweise einzeln zu steuern. Deshalb ist das eine Ventil als Bypass-Ventil und das andere als Frequenzventil ausgebildet.

Patentanspruch 6 gibt die technische Wirkung des Bypass-Ventils an.

Patentanspruch 7 gibt die technische Wirkung des Frequenzventils an.

Vorteilhafte Druckbereiche und Frequenzen für den erzeugten Luftdruck geben die Merkmale der Patentansprüche 8 und 9 an.

Die Fernbedienung nach Patentanspruch 10 erleichtert die Bedienung des Pumpsets und der Brustpumpe.

Die Ausbildung der Fernbedienung nach Patentanspruch 11 ermöglicht eine komfortablere Handhabung des Pumpsets.

Der Einsatz von Speichermitteln gemäß Patentanspruch 12 ermöglicht in vorteilhafter Weise, dass an die gleiche Brustpumpe verschiedene Pumpsets angeschlossen werden können. So kann eine Mehrzahl von Müttern jeweils ihr eigenes Pumpset verwenden und ein und dieselbe Brustpumpe liefert den individuell benötigten Luftdruck aufgrund der benutzerbezogenen Daten.

Im Folgenden wird eine bevorzugte Ausführungsform der Erfindung unter Bezugnahme auf die Figuren 1 bis 3 näher erläutert.

Dabei zeigen:
- Fig. 1:: ein Ausführungsbeispiel der erfindungsgemäßen Muttermilchpumpe mit Pumpset und Brustpumpe.
- Fig. 2a:: eine erste Ausführung eines Pumpsets im Schnitt.
- Fig. 2b:: eine zweite Ausführung eines Pumpsets im Schnitt.
- Fig. 2c:: eine dritte Ausführung eines Pumpsets im Schnitt.
- Fig. 3:: eine Brustpumpe im Schnitt mit Trennwand und Kompartimenten.

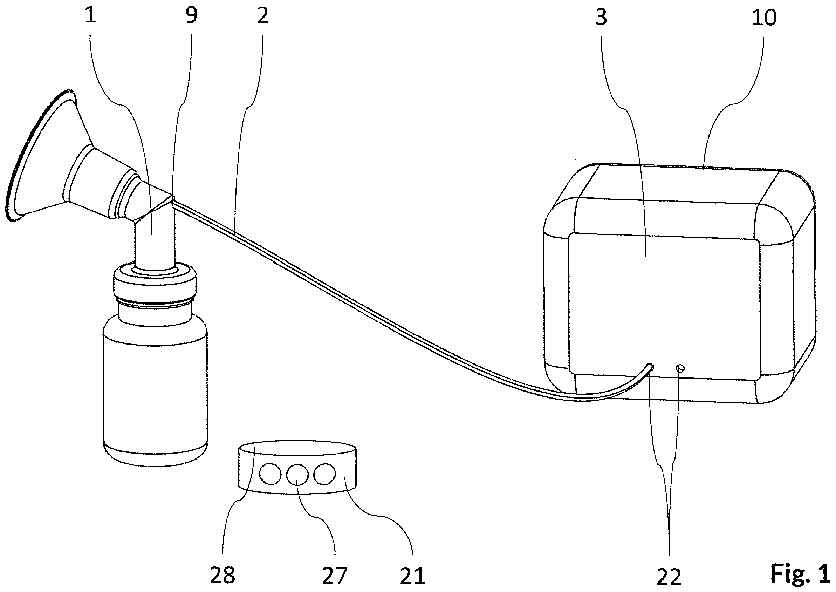

Figur 1 stellt ein Pumpset (1) und eine Brustpumpe (3) dar, verbunden mittels eines Luftschlauches (2). Dieser ist einerseits an einem dafür vorgesehenen Schlauchanschluss (22) in einem Gehäuse (10) der Brustpumpe (3), andererseits an einem Schlauchanschluss (9) des Pumpsets (1) angeschlossen. An einem möglicherweise ausgebildeten weiteren Schlauchanschluss (22) im Gehäuse (10) der Brustpumpe (3) kann ein weiteres, hier nicht dargestelltes, baugleiches Pumpset (1) mit dem baugleichen Luftschlauch (2) angeschlossen sein. Alternativ kann ein Luftschlauch (2) verwendet werden, der an nur einem Schlauchanschluss (22) im Gehäuse (10) der Brustpumpe (3) angeschlossen ist und gemeinsam zwei Pumpsets (1) mit Luft versorgt.

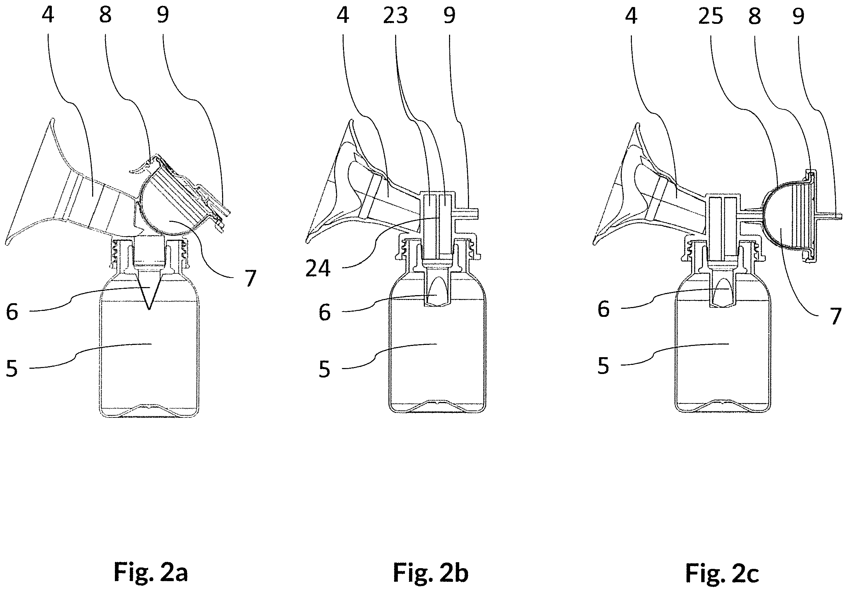

Figur 2a zeigt eine erste Ausführung eines Pumpsets (1) im Schnitt. Es besteht aus einer Brustglocke (4) mit trichterförmigem Ansatz. In diesen kann bei Bedarf ein Softeinsatz eingesetzt sein. Dieser ist gegebenenfalls durch einen Hygienedeckel verschließbar. Von unten ist eine Milchflasche (5) in die Brustglocke (4) eingeschraubt. Dafür hat sich ein weites Gewinde als besonders geeignet gezeigt, da seine Handhabung problemlos ist. Die eingeschraubte Milchflasche (5) hält ein Ventil (6) in der Gewindebohrung der Brustglocke (4).

Bei dieser Ausführung ist in der Brustglocke (4) eine Öffnung für eine Schutzmembran (7) vorgesehen, die eine hygienische Barriere bildet zwischen der Luft, die von der Brustpumpe (3) kommt und der abgepumpten Muttermilch. Diese Schutzmembran (7) ist von einem Verschlussdeckel (8) druckdicht abgedeckt. An diesem ist ein Schlauchanschluss (9) zum Aufstecken des Luftschlauches (2) angeformt.

Figur 2b zeigt eine zweite Ausführung eines Pumpsets (1) im Schnitt. Diese unterscheidet sich von der ersten dadurch, dass in der Brustglocke (4) zwei Kammern (23) ausgebildet sind, zwischen denen eine elastische Trennwand (24) vorhanden ist. Die in der Figur rechte Kammer (23) weist den Schlauchanschluss (9) auf, auf den der Luftschlauch (2) aufsteckbar ist.

Figur 2c zeigt eine dritte Ausführung eines Pumpsets (1) im Schnitt. Bei dieser sind die Schutzmembran (7) mit Verschlussdeckel (8) in einem separaten Gehäuse (25) untergebracht, welches auf die Brustglocke (4) aufgesteckt ist. Auch hier ist auf den Schlauchanschluss (9) des Verschlussdeckels (8) der Luftschlauch (2) aufsteckbar.

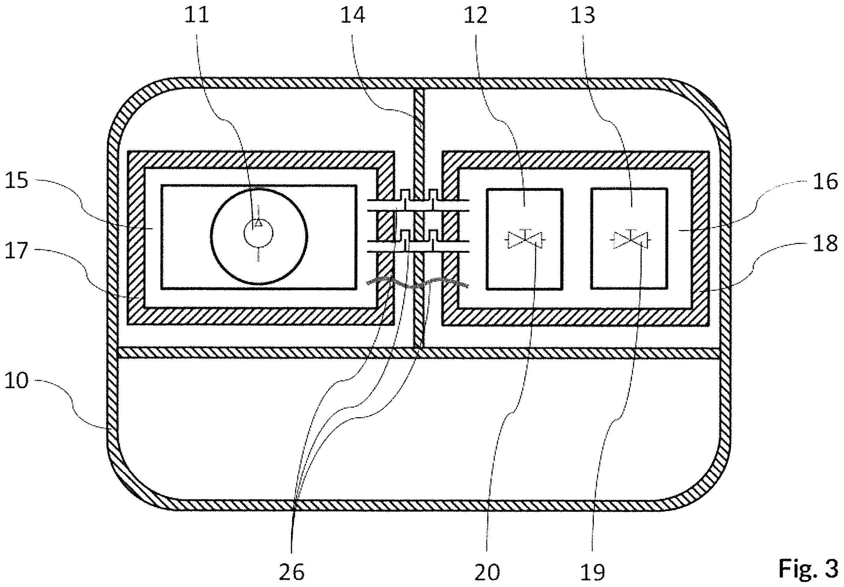

Die Schnittdarstellung der Brustpumpe (3) in Figur 3 zeigt das Gehäuse (10) mit einer in dieser eingebrachten Trennwand (14). In die dadurch entstandenen zwei Räume sind jeweils ein Kompartiment (15, 16) eingesteckt und arretiert.

Das eine Kompartiment (15) enthält eine pneumatische Pumpe (11), das andere Kompartiment (16) Ventile (12, 13). Die beiden Kompartimente (15, 16) sind elektrisch und pneumatisch verbunden. Dies erfolgt durch elektrische und pneumatische Verbindungsleitungen 26, die die in den Kompartimenten (15, 16) enthaltenen Ventile (12, 13) und die pneumatische Pumpe (11) schaltungstechnisch verbinden. Die elektrischen Verbindungsleitungen (26) dienen der Stromversorgung und der Weiterleitung der Steuersignale. Die pneumatischen Verbindungsleitungen (26) werden für Überdruck- und Unterdruckversorgung benötigt. Die Verbindungsleitungen (26) sind so flexibel ausgelegt, dass zwischen den Kompartimenten (15, 16) keine Schwingungen übertragen werden.

Wie weiterhin in Figur 3 gezeigt, ist das Kompartiment (15) mit einer Dämmung (17) ausgekleidet. Die darin vorhandene pneumatische Pumpe (11), die gleichzeitig Über- und Unterdruck erzeugt, wird mit konstanter Drehzahl betrieben. So wird nur ein enger Frequenzbereich oder ein schmales Frequenzband des Schalles erzeugt. Als Dämmung (17) für diesen Schall kommt ein technischer Akustikabsorber zur Anwendung, dessen Spezifikationen an den auftretenden Frequenzbereich angepasst sind. Auf diese Weise wird eine optimale Schalldämmung erzielt.

Das andere Kompartiment (16) enthält die Ventile (12, 13). Dabei handelt es sich um ein Bypass-Ventil (19) und ein Frequenzventil (20). Das Bypass-Ventil (19) variiert durch sein Öffnen und Schließen einer Öffnung zur Umgebungsluft den von der pneumatischen Pumpe (11) bereitgestellten konstanten Unterdruck, während der Stimulationsphase auf bis zu ±40 kPa, während der Abpumpphase auf bis zu ±50 kPa. Diese Druckwerte stellen Maximalwerte dar. Das Frequenzventil (20) steuert ausschließlich die Frequenz des zum Pumpset (1) geleiteten Luftdruckes, während der Stimulationsphase mit einer Frequenz von 80-140 Zyklen/min bei ±40 kPa (Vakuumbereich am Anwendungsteil Pumpset -5 kPa bis -20 kPa), während der Abpumpphase mit einer Frequenz von 20-80 Zyklen/min bei ±50 kPa (Vakuumbereich am Anwendungsteil Pumpset -10 kPa bis -33 kPa). Dafür ist das Frequenzventil (20) einerseits dazu in der Lage, zwischen Über- und Unterdruck umzuschalten und andererseits die zeitliche Länge des jeweiligen Über- und Unterdrucks zu schalten. Auf diese Weise gelangt der gewünschte Luftdruck zum Pumpset (1).

Diese Ventile erzeugen im Betrieb einen eigenen, signifikanten Frequenzbereich des Schalles. Zur optimalen Schalldämmung ist das Kompartiment (16) mit einer angepassten Schalldämmung ausgekleidet. Als Dämmung (18) für diesen Schall kommt ein anderer technischer Akustikabsorber zur Anwendung, dessen Spezifikationen an den auftretenden Frequenzbereich angepasst sind.

Mittels einer Fernbedienung (21) (s. Figur 1) sind benutzerbezogene Daten für individuelle Steuervorgänge einstellbar, wie zeitliche Länge der Stimulationsphase und Länge der Abpumpphase. Auch können die Luftdrücke und die Frequenzen bestimmt werden, die angewendet werden sollen. Dazu sind in der Fernbedienung (21) Bedienungsmittel (27) vorhanden, welche auf ein Speichermittel wirken, das die benutzerbezogenen Daten enthält. Die Fernbedienung (21) kommuniziert mit der Brustpumpe (3) mittels Sendeeinheit und Empfangseinheit. Das Speichermittel enthält zudem individuelle Daten der Nutzerin. Damit ist eine an die Nutzerin angepasste Einstellung von Frequenzen und Druckverläufen während der Stimulationsphase und der Abpumpphase sichergestellt. Die Fernbedienung (21) und der Brustpumpe (3) sind so von verschiedenen Nutzerinnen verwendbar. Da aus hygienischen Gründen für jede Anwenderin ein neues Pumpset (1) zu verwenden ist, kann dieses somit ohne Schwierigkeiten an die Brustpumpe (3) angeschlossen werden.

Die in Figur 1 gezeigte Fernbedienung (21) verfügt über eine Halterung (28) für die Arretierung des Pumpset (1).

Das Speichermittel, z.B. ein RFID-Chip, erlaubt die Anpassung der Einstellungen an die verschiedenen Nutzerinnen auf diesem einfachen Weg. Diverse Stimulations- und Abpumpprogramme können auf dem Speichermittel vorhinterlegt sein. Der Wechsel zwischen den Pumpphasen erfolgt automatisch und fließend.

## Patentansprüche

1. Muttermilchpumpe bestehend aus einem oder zwei Pumpsets (1) die jeweils mit einem oder einem gemeinsamen Luftschlauch (2) mit einer Brustpumpe (3) verbunden sind, wobei das Pumpset (1) aus einer Brustglocke (4), aus einer Milchflasche (5), die von unten in die Brustglocke (4) einschraubbar ist, aus einem Ventil (6), das zwischen Milchflasche (5) und Brustglocke (4) angeordnet ist, und aus einer Schutzmembran (7), die entweder an der Brustglocke (4) angebaut ist oder als externes Bauteil angebrachten werden kann, und die von einem Verschlussdeckel (8) verschließbar ist, wobei der Verschlussdeckel (8) über einen Schlauchanschluss (9) zum Anschließen des Luftschlauches (2) verfügt, der mit der Brustpumpe (3) verbunden ist, besteht und die Brustpumpe (3) ein Gehäuse (10) aufweist, in dem mindestens eine pneumatische Pumpe (11) und Ventile (12, 13) eingebaut sind, wobei in das Gehäuse (10) eine Trennwand (14) eingefügt ist und in die dadurch gebildeten Räume jeweils ein Kompartiment (15, 16) mit eigener Dämmung (17, 18) eingebracht ist, **dadurch gekennzeichnet, dass** das eine Kompartiment (15) die pneumatische Pumpe (11) und das andere Kompartiment (16) die Ventile (12, 13) enthält.

2. Muttermilchpumpe nach Patentanspruch 1,
**dadurch gekennzeichnet,**
**dass** das Kompartiment (15) eine an die pneumatische Pumpe (11) baulich und akustisch angepasste Dämmung (17) aufweist.

3. Muttermilchpumpe nach Patentanspruch 1 oder 2,
**dadurch gekennzeichnet,**
**dass** das Kompartiment (16) eine an die Ventile (12, 13) baulich und akustisch angepasste Dämmung (18) aufweist.

4. Muttermilchpumpe nach einem der Patentansprüche 1 bis 3,
**dadurch gekennzeichnet,**
**dass** die Kompartimente (15, 16) elektrisch und pneumatisch verbunden sind.

5. Muttermilchpumpe nach einem der vorhergehenden Patentansprüche,
**dadurch gekennzeichnet,**
**dass** die Ventile (12, 13) ein Bypass-Ventil (19) und ein Frequenzventil (20) sind.

6. Muttermilchpumpe nach Patentanspruch 5,
**dadurch gekennzeichnet,**
**dass** das Bypass-Ventil (19) den Wert des zum Pumpset (1) geleiteten Luftdrucks regelt.

7. Muttermilchpumpe nach Patentanspruch 5,
**dadurch gekennzeichnet,**
**dass** das Frequenzventil (20) eine Frequenz des zum Pumpset (1) geleiteten Luftdrucks schaltet.

8. Muttermilchpumpe nach Patentanspruch 6,
**dadurch gekennzeichnet,**
**dass** das Bypass-Ventil (19) das Vakuum der Brustpumpe (3) auf 0 bis -50 kPa einstellt.

9. Muttermilchpumpe nach Patentanspruch 7,
**dadurch gekennzeichnet,**
**dass** das Frequenzventil (20) mit einer Frequenz von 20-140 Zyklen/min das Vakuum und den Druck schaltet.

10. Muttermilchpumpe nach einem der vorhergehenden Patentansprüche,
**dadurch gekennzeichnet,**
**dass** eine Fernbedienung (21) vorhanden ist, die die Betriebsmodi der Milchpumpe steuert, wobei insbesondere das Gehäuse (10) die Fernbedienung (21) aufweist.

11. Muttermilchpumpe nach Patentanspruch 10,
**dadurch gekennzeichnet,**
**dass** an der Fernbedienung (21) eine Halterung (28) für das Pumpset (1) ausgebildet ist.

12. Muttermilchpumpe nach Patentanspruch 10 oder 11,
**dadurch gekennzeichnet,**
**dass** die Fernbedienung (21) Speichermittel und/oder Kommunikationsmittel zur Aufnahme und Übertragung benutzerbezogener Daten für individuell eingestellte Steuervorgänge enthält.

13. Muttermilchpumpe nach einem der vorhergehenden Ansprüche,
**dadurch gekennzeichnet,**
**dass** die Kompartimente (15, 16) durch flexible Verbindungsleitungen (26) elektrisch und pneumatisch verbunden sind.

## Claims

1. Breast milk pump consisting of one or two pump sets (1), each of which is connected to a breast pump (3) via one or a common air hose (2), the pump set (1) consisting of a breast cup (4), a milk bottle (5), which can be screwed into the breast cup (4) from below, a valve (6), which is arranged between the milk bottle (5) and the breast cup (4), and a protective membrane (7), which is either attached to the breast cup (4) or can be attached as an external component and which can be closed by a closure cover (8), the closure cover (8) having a hose connection (9) for connecting the air hose (2) which is connected to the breast pump (3), and the breast pump (3) comprising a housing (10) in which at least one pneumatic pump (11) and valves (12, 13) are incorporated, a partition (14) being inserted into the housing (10) and a compartment (15, 16) having its own insulation (17, 18) being introduced into each of the spaces thus formed, **characterized in that** one compartment (15) contains the pneumatic pump (11) and the other compartment (16) contains the valves (12, 13).

2. Breast milk pump according to claim 1, **characterized in that** the compartment (15) has insulation (17) which is structurally and acoustically adapted to the pneumatic pump (11).

3. Breast milk pump according to either claim 1 or claim 2, **characterized in that** the compartment (16) has insulation (18) which is structurally and acoustically adapted to the valves (12, 13).

4. Breast milk pump according to any of claims 1 to 3, **characterized in that** the compartments (15, 16) are connected electrically and pneumatically.

5. Breast milk pump according to any of the preceding claims, **characterized in that** the valves (12, 13) are a bypass valve (19) and a frequency valve (20).

6. Breast milk pump according to claim 5, **characterized in that** the bypass valve (19) controls the value of the air pressure supplied to the pump set (1).

7. Breast milk pump according to claim 5, **characterized in that** the frequency valve (20) switches a frequency of the air pressure supplied to the pump set (1).

8. Breast milk pump according to claim 6, **characterized in that** the bypass valve (19) sets the vacuum of the breast pump (3) to 0 to -50 kPa.

9. Breast milk pump according to claim 7, **characterized in that** the frequency valve (20) switches the vacuum and the pressure at a frequency of 20-140 cycles/min.

10. Breast milk pump according to any of the preceding claims, **characterized in that** a remote control (21) is provided which controls the operating modes of the milk pump, the housing (10) in particular having the remote control (21).

11. Breast milk pump according to claim 10, **characterized in that** a holder (28) for the pump set (1) is formed on the remote control (21).

12. Breast milk pump according to either claim 10 or claim 11, **characterized in that** the remote control (21) contains storage means and/or communication means for receiving and transmitting user-related data for individually set control processes.

13. Breast milk pump according to any of the preceding claims, **characterized in that** the compartments (15, 16) are connected electrically and pneumatically by flexible connecting lines (26).

## Revendications

1. Pompe à lait maternel composée d'un ou de deux ensembles de pompe (1), chacun étant relié à un tire-lait (3) au moyen d'un tuyau à air (commun) (2), l'ensemble de pompe (1) se composant d'une téterelle (4), d'un biberon (5) qui peut être vissé dans la téterelle (4) par le bas, d'une soupape (6) qui est disposée entre le biberon (5) et la téterelle (4), et d'une membrane protectrice (7) qui est soit fixée à la téterelle (4), soit peut être installée comme composant externe, et qui peut être fermée par un couvercle de fermeture (8), le couvercle de fermeture (8) disposant d'un raccord de tuyau (9) permettant de raccorder le tuyau à air (2) qui est relié au tire-lait (3), et le tire-lait (3) présentant un carter (10) dans lequel sont installés au moins une pompe pneumatique (11) et des soupapes (12, 13), une cloison (14) étant placée dans le carter (10) et respectivement un compartiment (15, 16), comportant chacun sa propre isolation (17, 18), étant inséré dans les espaces ainsi formés, **caractérisée en ce qu'**un compartiment (15) contient la pompe pneumatique (11) et **en ce que** l'autre compartiment (16) contient les soupapes (12, 13).

2. Pompe à lait maternel selon la revendication 1,
**caractérisée en ce**
**que** le compartiment (15) présente une isolation (17) structurellement et acoustiquement adaptée à la pompe pneumatique (11).

3. Pompe à lait maternel selon la revendication 1 ou 2,
**caractérisée en ce**
**que** le compartiment (16) présente une isolation (18) structurellement et acoustiquement adaptée aux soupapes (12, 13).

4. Pompe à lait maternel selon l'une des revendications 1 à 3,
**caractérisée en ce**
**que** les compartiments (15, 16) sont reliés électriquement et pneumatiquement.

5. Pompe à lait maternel selon l'une des revendications précédentes,
**caractérisée en ce**
**que** les soupapes (12, 13) sont une soupape de dérivation (19) et une soupape de fréquence (20).

6. Pompe à lait maternel selon la revendication 5,
**caractérisée en ce**
**que** la soupape de dérivation (19) régule la valeur de la pression d'air amenée à l'ensemble de pompe (1).

7. Pompe à lait maternel selon la revendication 5,
**caractérisée en ce**
**que** la soupape de fréquence (20) commute une fréquence de la pression d'air amenée à l'ensemble de pompe (1).

8. Pompe à lait maternel selon la revendication 6,
**caractérisée en ce**
**que** la soupape de dérivation (19) règle le vide du tire-lait (3) entre 0 et -50 kPa.

9. Pompe à lait maternel selon la revendication 7,
**caractérisée en ce**
**que** la soupape de fréquence (20) commute le vide et la pression avec une fréquence de 20 à 140 cycles/min.

10. Pompe à lait maternel selon l'une des revendications précédentes,
**caractérisée en ce**
**qu'**une télécommande (21) qui commande les modes de fonctionnement de la pompe à lait est prévue, le carter (10) présentant en particulier la télécommande (21).

11. Pompe à lait maternel selon la revendication 10,
**caractérisée en ce**
**qu'**un support (28) destiné à l'ensemble de pompe (1) est formé sur la télécommande (21).

12. Pompe à lait maternel selon la revendication 10 ou 11,
**caractérisée en ce**
**que** la télécommande (21) contient des moyens de stockage et/ou des moyens de communication permettant de recevoir et transmettre des données relatives à l'utilisateur destinées à des processus de commande réglés individuellement.

13. Pompe à lait maternel selon l'une des revendications précédentes,
**caractérisée en ce**
**que** les compartiments (15, 16) sont reliés électriquement et pneumatiquement par des conduites de liaison flexibles (26).
